# EUROPEAN PATENT APPLICATION

(11) **EP 4 124 863 A1**
(43) Date of publication of application: **01.02.2023**
(21) Application number: 21905068.9
(22) Date of filing: 04.08.2021
(51) Int. Cl.: G01N 33/543, G01N 33/532

(54) **CHEMILUMINESCENCE IMMUNOREAGENT, LYOPHILIZED MICROSPHERE, AND PREPARATION METHOD FOR LYOPHILIZED MICROSPHERE**

(30) Priority: 18.12.2020 CN 202011500680
(71) Applicant: Anbio (Xiamen) Biotechnology Co., Ltd., Xiamen, Fujian 361000 (CN)
(72) Inventor: CHEN, Zhujin, Xiamen, Fujian 361026 (CN); CHENG, Tao, Xiamen, Fujian 361026 (CN); ZHANG, Jieli, Xiamen, Fujian 361026 (CN); ZHANG, Linhua, Xiamen, Fujian 361026 (CN); HU, Xiao, Xiamen, Fujian 361026 (CN); LEI, Yang, Xiamen, Fujian 361026 (CN)
(74) Representative: Bandpay & Greuter
(86) International application number: PCT/CN2021/110438
(87) International publication number: WO 2022/127135

(57) **Abstract**

The present invention provides a chemiluminescent immunoreagent, which includes a magnetic particle coating raw material with a magnetic particle as a solid phase carrier coupled with an antigen or an antibody and other raw materials; and an acridinium ester marking raw material with an acridinium ester as a luminescent signal marker marking an antigen or an antibody and other raw materials. The present invention further provides freeze-dried microspheres of the chemiluminescent immunoreagent, which further includes a reagent storage agent that protects the stability of the reagent and provides an environment required for an immunoreaction, wherein the reagent storage agent includes a freeze-drying protectant, PEG20000 and an antioxidant. The present invention further provides a method for preparing freeze-dried microspheres of a chemiluminescent immunoreagent, which includes the following steps: preparing a chemiluminescent immunoreagent from a magnetic particle coating raw material and an acridinium ester marking raw material; adding a reagent storage agent for protecting the stability of a reagent; freeze-drying the chemiluminescent immunoreagent with liquid nitrogen to form frozen microspheres; and transferring the frozen microspheres into a freeze dryer, freeze-drying the frozen microspheres, charging a protective gas, and sub-packaging and storing the freeze-dried microspheres. The present invention solves the problem that luminescent immunoreagents can't be stored and transported at normal temperature.

## Description

### Technical Field

The present invention relates to the technical field of chemiluminescent immunoreagents, in particular to a chemiluminescent immunoreagent, freeze-dried microspheres, and a preparation method of the freeze-dried microspheres.

### Background Art

In recent years, luminescent immunoreagents have been widely applied in food testing, pathogen detection and oncogene diagnosis, etc. However, there are rigorous requirements for the conditions of storage and transportation of luminescent immunoreagents. Usually, luminescent immunoreagents must be stored at 2-8°C, and transported through a cold chain. If those conditions are not met, the performance of the luminescent immunoreagents will be severely affected, and even the reagents may fail.

Presently, many instant detection products have been put into the Chinese market. However, it is not easy to use existing luminescent immunoreagents to isolate and detect the substances to be detected.

### Contents of the Invention

An object of the present invention is to provide a chemiluminescent immunoreagent, freeze-dried microspheres of the chemiluminescent immunoreagent, and a preparation method of the freeze-dried microspheres, so as to solve the problem that it is not easy to isolate and detect the substances to be detected with luminescent immunoreagents in the prior art.

The above object of the present invention is attained with the following technical schemes:
A reagent, comprising:
a magnetic particle coating raw material with a magnetic particle as a solid phase carrier coupled with an antigen or an antibody and other raw materials; and
an acridinium ester marking raw material with an acridinium ester as a luminescent signal marker marking an antigen or an antibody and other raw materials.

A method for preparing freeze-dried microspheres of a chemiluminescent immunoreagent that further comprises a reagent storage agent that reduces various stress damages to the reagent during freeze-drying, protects the reagent against degeneration and deactivation, thereby protects the stability of the reagent, wherein the reagent storage agent comprises a freeze-drying protectant, PEG20000, and an antioxidant.

Furthermore, the freeze-drying protectant comprises mannitol, trehalose, casein, surfactant, gelatin, preservative, and buffer solution TBS.

Furthermore, based on the total mass percentage of the freeze-drying protectant, which is regarded as 100%, the mass percentages of the components are as follows: mannitol: 2% - 10%; trehalose: 5% - 20%; casein: 0.5% - 2%; surfactant: 0.05% - 0.5%; gelatin: 0.05% - 1%; preservative: 0.1% - 1%; and buffer solution TBS: 65.5% - 92.3%.

Furthermore, the buffer solution TBS is made of 10-50 mm TRIS-HCL and 0.85% NaCl, and the pH of the buffer solution is neutral, specifically pH=7.0-7.4.

Furthermore, the surfactant is Tween 20, Tween 80 or Triton x 100.

Furthermore, the antioxidant is a mixture of sodium thiosulfate and EDTA-2Na mixed at 1: 1 mix ratio.

A method for preparing freeze-dried microspheres of a chemiluminescent immunoreagent, comprising the following steps:
S1: preparing a chemiluminescent immunoreagent from a magnetic particle coating raw material and an acridinium ester marking raw material, wherein a magnetic particle is used as a solid phase carrier, and an acridinium ester is used as a luminescent signal marker;
S2: adding a reagent storage agent to the prepared chemiluminescent immunoreagent to protect the stability of the reagent;
S3: freeze-drying the chemiluminescent immunoreagent with liquid nitrogen to form freeze-dried pellets, freezing the frozen pellets individually to form frozen microspheres, and packaging the frozen microspheres in individual packages for assay;
S4: transferring the frozen microspheres into a freeze dryer, charging a protective gas after freeze-drying, and sub-packaging and storing the freeze-dried microspheres, so as to solve the problem that the existing chemiluminescence reagents cannot be stored and transported at normal temperature.

### Beneficial Technical Effects

1. The chemiluminescent immunoreagent is prepared from a magnetic particle coating raw material and an acridinium ester marking raw material, magnetic particles are used as solid phase carriers, an acridinium ester is used as a luminescent signal marker, the reagent reacts with the sample to form an immune complex of the magnetic particle coupling raw material, the substance to be detected, and the acridinium ester marking raw material, the immune complex is separated by washing under the effect of an applied magnetic field, and the complex is luminescent under the action of an excitation liquid, thereby the detection of the substance to be detected is realized;
2. By adding a reagent storage agent, the reagent is protected in the environment of temperature, humidity and PH value, so that the prepared luminescent immunoreagent can be freeze-dried to form pellets, and then the samples are frozen individually to form frozen microspheres, which are sub-packaged individually for assay;
3. The pellets freeze-dried with liquid nitrogen are frozen individually into frozen microspheres, and then the freeze-dried microspheres are transferred into a freeze drier and freeze-dried, then a protective gas is charged and the freeze-dried microspheres are sub-packaged and stored; thus, the problem that the existing chemiluminescent immunoreagents can't be stored and transported at normal temperature is solved.

### Embodiments

To make the technical problem, technical scheme and benefits of the present invention understood more clearly, hereunder the present invention will be further detailed in embodiments. It should be understood that the embodiments described herein are only intended to explain the present invention, but shall not be deemed as constituting any limitation to the present invention.

A chemiluminescent immunoreagent disclosed in the present invention comprises:
a magnetic particle coating raw material with a magnetic particle as a solid phase carrier coupled with an antigen or an antibody and other raw materials; and
an acridinium ester marking raw material with an acridinium ester as a luminescent signal marker marking an antigen or an antibody and other raw materials.

A method for preparing freeze-dried microspheres of a chemiluminescent immunoreagent that comprises a magnetic particle coating raw material, an acridinium ester marking raw material, and a reagent storage agent for reducing various stress damages to the reagent during freeze-drying, protecting the reagent against degeneration and deactivation, and protecting the stability of the reagent.

The reagent storage agent comprises a freeze-drying protectant, PEG20000, and an antioxidant.

The freeze-drying protectant comprises mannitol, trehalose, casein, surfactant, gelatin, preservative, and buffer solution TBS.

In the freeze-drying protectant:
The mannitol is used as a freeze-drying filler, has no hygroscopicity, can achieve rapid freeze-drying, can prevent the active components from sublimating with water vapour, and can shape the active components;
The trehalose plays the role of a cryoprotectant in the freezing process and the role of a dehydration protectant in the drying process;
The casein protects the proteins, serves as a filler in the dehydration and drying process, and controls the background and eliminates false positive in the immunoreagent;
The surfactant is Tween 20, Tween 80 or Triton x 100, which reduces the freezing and dehydration deformation resulted from the ice-water interface tension in the freeze-drying process, attains the effects of a wetting agent and a refolding agent for the active components in the rehydration process, and reduces non-specific reactions in the immunoreaction process at the same time;
The gelatin serves as a freeze-drying filler, and can block the sites in the immunoreaction, improve the stability of the proteins, and eliminate non-specific reactions;
The preservative is Proclin 300, which has broad-spectrum bacteriostasis and high biocompatibility.
The PEG20000 serves as a cryoprotectant and a dehydration protectant, and can accelerate the reaction and improve the sensitivity of the reagent in the immunoreaction;
The antioxidants sodium thiosulfate and EDTA-2Na prevent the reagent from oxidative deterioration during freeze-drying and storage.

A method for preparing freeze-dried microspheres of a chemiluminescent immunoreagent, comprising the following steps:
S1: preparing a chemiluminescent immunoreagent from a magnetic particle coating raw material and an acridinium ester marking raw material, wherein a magnetic particle is used as a solid phase carrier, and an acridinium ester is used as a luminescent signal marker;
S2: adding a reagent storage agent to the prepared chemiluminescent immunoreagent to protect the stability of the reagent;
S3: freeze-drying the chemiluminescent immunoreagent with liquid nitrogen to form freeze-dried pellets, freezing the frozen pellets individually to form frozen microspheres, and packaging the frozen microspheres in individual packages for assay;
S4: transferring the frozen microspheres into a freeze dryer, filling a protective gas after freeze drying, and sub-packaging and storing the freeze-dried microspheres, so as to solve the problem that the existing chemiluminescence reagents cannot be stored and transported at normal temperature.

While the present invention is described above in embodiments with reference to the accompanying drawings, apparently the specific implementation of the present invention is not limited to those embodiments. Any non-substantive modification made on the basis of the ideal of the method and the technical scheme of the present invention or any direct application of the ideal and technical scheme of the present invention for other purposes without any modification shall be deemed as falling in the scope of protection of the present invention.

## Claims

1. A chemiluminescent immunoreagent, comprising:
a magnetic particle coating raw material with a magnetic particle as a solid phase carrier coupled with an antigen or an antibody and other raw materials; and
an acridinium ester marking raw material with an acridinium ester as a luminescent signal marker marking an antigen or an antibody and other raw materials.

2. Freeze-dried microspheres comprising the chemiluminescent immunoreagent according to claim 1, further comprising a reagent storage agent that reduces various stress damages to the reagent during freeze-drying, protects the reagent against degeneration and deactivation, thereby protects the stability of the reagent, wherein the reagent storage agent comprises a freeze-drying protectant, PEG20000, and an antioxidant.

3. The freeze-dried microspheres according to claim 2, wherein the freeze-drying protectant comprises mannitol, trehalose, casein, surfactant, gelatin, preservative, and buffer solution TBS.

4. The freeze-dried microspheres according to claim 2, wherein based on the total mass percentage of the freeze-drying protectant, which is regarded as 100%, the mass percentages of the components are as follows: mannitol: 2% - 10%; trehalose: 5% - 20%; casein: 0.5% - 2%; surfactant: 0.05% - 0.5%; gelatin: 0.05% - 1%; preservative: 0.1% - 1%; and buffer solution TBS: 65.5% - 92.3%.

5. The freeze-dried microspheres according to claim 3 or 4, wherein the buffer solution TBS is made of 10-50 mm TRIS-HCL and 0.85% NaCl, and the pH of the buffer solution is neutral, specifically pH=7.0-7.4.

6. The freeze-dried microspheres according to claim 2, wherein the surfactant is Tween 20, Tween 80 or Triton x 100.

7. The freeze-dried microspheres according to claim 2, wherein the antioxidant is a mixture of sodium thiosulfate and EDTA-2Na mixed at 1:1 mix ratio.

8. The freeze-dried microspheres according to any of claim 2-7, wherein a method for preparing the freeze-dried microspheres of a chemiluminescent immunoreagent comprises the following steps:
S1: preparing a chemiluminescent immunoreagent from a magnetic particle coating raw material and an acridinium ester marking raw material, wherein a magnetic particle is used as a solid phase carrier, and an acridinium ester is used as a luminescent signal marker;
S2: adding a reagent storage agent to the prepared chemiluminescent immunoreagent to protect the stability of the reagent;
S3: freeze-drying the chemiluminescent immunoreagent with liquid nitrogen to form freeze-dried pellets, freezing the frozen pellets individually to form frozen microspheres, and packaging the frozen microspheres in individual packages for assay;
S4: transferring the frozen microspheres into a freeze dryer, charging a protective gas after freeze-drying, and sub-packaging and storing the freeze-dried microspheres, so as to solve the problem that the existing chemiluminescence reagents cannot be stored and transported at normal temperature.
